# EUROPEAN PATENT SPECIFICATION

(11) **EP 3 315 572 B1**
(45) Date of publication and mention of the grant of the patent: **12.05.2021**
(21) Application number: 16814470.7
(22) Date of filing: 24.06.2016
(51) Int. Cl.: C09J 123/00, B32B 25/08, B32B 25/14, B32B 27/00, C09J 11/06, A41D 13/11, A61F 13/49, C09J 123/14

(54) **HOT-MELT PRESSURE-SENSITIVE ADHESIVE, STRETCHABLE LAMINATE, AND ARTICLE**
HAFTSCHMELZKLEBSTOFF, DEHNBARES LAMINAT UND ARTIKEL
ADHÉSIF THERMOFUSIBLE SENSIBLE À LA PRESSION, STRATIFIÉ ÉTIRABLE ET ARTICLE

(30) Priority: 26.06.2015 JP 2015128967
(43) Date of publication of application: 02.05.2018
(73) Proprietor: Nitto Denko Corporation, Ibaraki-shi, Osaka 567-8680 (JP)
(72) Inventor: IKISHIMA, Shinsuke, Ibaraki-shi Osaka 567-8680 (JP); UCHIDA, Shou, Ibaraki-shi Osaka 567-8680 (JP); TAKEDA, Kohei, Ibaraki-shi Osaka 567-8680 (JP)
(74) Representative: Müller-Boré & Partner Patentanwälte PartG mbB
(86) International application number: PCT/JP2016/068757
(87) International publication number: WO 2016/208701

(56) References cited:
- JP-A- 2003 507 561
- JP-A- 2008 524 431
- JP-A- 2013 064 055
- JP-A- 2014 527 559
- JP-A- 2015 077 784
- JP-B2- S 631 352
- US-A1- 2014 147 669

## Description

The present invention relates to a hot-melt pressure-sensitive adhesive, a stretchable laminate, and an article, and more specifically, to a hot-melt pressure-sensitive adhesive, a stretchable laminate having the hot-melt pressure-sensitive adhesive, and an article including the stretchable laminate.

Various stretchable laminates have been proposed as members for articles such as sanitary articles, for example, a diaper and a mask (see, for example, Patent Literatures 1 and 2).

A stretchable laminate including a non-woven fabric layer on at least one side of an elastomer layer has been proposed as such member. In such stretchable laminate, the elastomer layer and the non-woven fabric layer are generally bonded to each other with an adhesive or a pressure-sensitive adhesive.

Recently, a hot-melt pressure-sensitive adhesive has been frequently adopted as such adhesive or pressure-sensitive adhesive. However, a related-art hot-melt pressure-sensitive adhesive produces a non-negligible level of odor, and hence the odor has been a problem particularly at the time of the adoption of the pressure-sensitive adhesive in a stretchable laminate that can be used in an article such as a sanitary material.

In addition, the hot-melt pressure-sensitive adhesive is required to be capable of being easily applied in a relatively low temperature region from about 140°C to about 160°C. However, the related-art hot-melt pressure-sensitive adhesive involves a problem in that its melt viscosity increases in the relatively low temperature region from about 140°C to about 160°C, and hence it becomes difficult to evenly apply the pressure-sensitive adhesive.

### Citation List

### Patent Literature

[PTL 1] JP 2012-187857 A
[PTL 2] JP 3830818 B2
[PTL 3] JP S63-1352 B2 describes hot-melt pressure sensitive adhesives.
[PTL 4] US 2014/0147669 A1 describes adhesives and use thereof.

The present invention has been made to solve the problems of the related art, and an object of the present invention is to provide a hot-melt pressure-sensitive adhesive that is sufficiently suppressed in odor and can be evenly applied in a relatively low temperature region. Another object of the present invention is to provide a stretchable laminate having such hot-melt pressure-sensitive adhesive. Still another object of the present invention is to provide an article including such stretchable laminate.

According to one embodiment of the present invention, there is provided a hot-melt pressure-sensitive adhesive, including:
a Ziegler-Natta-based olefin resin;
a metallocene-based olefin resin;
a hydrocarbon-based tackifier; and
an oil component,
in which the hot-melt pressure-sensitive adhesive has:
   an adhesive strength at 23°C, a peel angle of 90°, and a peel rate of 300 mm/min on a polypropylene spunbonded non-woven fabric having a basis weight of 19 g/m² of 200 g/25 mm or more;
   an adhesive strength at 23°C, a peel angle of 90°, and a peel rate of 300 mm/min on a stainless-steel plate of 700 g/25 mm or more;
   a melt viscosity at 160°C of 10,000 mPa·s or less;
   a melt viscosity at 140°C of 15,000 mPa·s or less; and
   a softening point temperature of 100°C or less,
   wherein the content of the metallocene-based olefin resin in the hot-melt pressure-sensitive adhesive is from 8 wt% to 0.1 wt%,
   and wherein the content of the hydrocarbon-based tackifier in the hot-melt pressure-sensitive adhesive is from 60 wt% to 30 wt%.

In one embodiment, the Ziegler-Natta-based olefin resin is free of an aromatic structure.

In one embodiment, a content of the Ziegler-Natta-based olefin resin in the hot-melt pressure-sensitive adhesive is from 70 wt% to 20 wt%.

In one embodiment, the metallocene-based olefin resin is free of an aromatic structure.

In one embodiment, the content of the metallocene-based olefin resin in the hot-melt pressure-sensitive adhesive is from 5 wt% to 1 wt%.

In one embodiment, the hydrocarbon-based tackifier is free of an aromatic structure.

In one embodiment, a content of the oil component in the hot-melt pressure-sensitive adhesive is from 40 wt% to 5 wt%.

In one embodiment, the oil component includes a paraffin oil.

According to another embodiment of the present invention, there is provided a stretchable laminate, including:
an elastomer layer; and
a non-woven fabric layer arranged on at least one side of the elastomer layer,
in which the stretchable laminate has the hot-melt pressure-sensitive adhesive according to the embodiment of the present invention between the elastomer layer and the non-woven fabric layer.

In one embodiment, a main component of the elastomer layer includes an elastomer resin and the elastomer resin includes an olefin-based elastomer.

In one embodiment, the olefin-based elastomer has a density of from 0.890 g/cm³ to 0.830 g/cm³.

In one embodiment, the olefin-based elastomer has a MFR at 230°C and 2.16 kgf of from 1.0 g/10 min to 25.0 g/10 min.

In one embodiment, a non-woven fabric forming the non-woven fabric layer includes a carded non-woven fabric.

According to still another embodiment of the present invention, there is provided an article, including the stretchable laminate according to the embodiment of the present invention.

According to the present invention, the hot-melt pressure-sensitive adhesive that is sufficiently suppressed in odor and can be evenly applied in a relatively low temperature region can be provided. In addition, the stretchable laminate having such hot-melt pressure-sensitive adhesive can be provided. Further, the article including such stretchable laminate can be provided.

FIG. 1 is a schematic sectional view of a stretchable laminate according to a preferred embodiment of the present invention.
FIG. 2 is a schematic view for illustrating another stretchable laminate according to a preferred embodiment of the present invention.
FIG. 3 is a schematic view of a state in which the top of a non-woven fabric is coated with a hot-melt pressure-sensitive adhesive in a stripe manner in the flow direction of a production line, the state being viewed from above.

### <<<<Hot-melt Pressure-sensitive Adhesive>>>>

A hot-melt pressure-sensitive adhesive of the present invention includes a Ziegler-Natta-based olefin resin, a metallocene-based olefin resin, a hydrocarbon-based tackifier, and an oil component.

The Ziegler-Natta-based olefin resin is an olefin-based resin obtained by polymerization involving using a Ziegler-Natta catalyst. The number of kinds of the Ziegler-Natta-based olefin resins may be only one, or may be two or more.

Any appropriate olefin-based resin may be adopted as the Ziegler-Natta-based olefin resin to the extent that the effects of the present invention are not impaired as long as the olefin-based resin is obtained by polymerization involving using a Ziegler-Natta catalyst. Preferred examples of such Ziegler-Natta-based olefin resin include a homopolymer of an α-olefin and a copolymer of two or more kinds of α-olefins. The α-olefin is preferably an α-olefin having 2 to 20 carbon atoms. Examples of such α-olefin include ethylene, propylene, 1-butene, 1-pentene, 1-hexene, 1-heptene, 1-octene, and 4-methyl-1-pentene. The Ziegler-Natta-based olefin resin is more preferably a Ziegler-Natta-based olefin resin free of an aromatic structure. The adoption of the Ziegler-Natta-based olefin resin free of an aromatic structure can provide a hot-melt pressure-sensitive adhesive more sufficiently suppressed in odor. The Ziegler-Natta-based olefin resin is preferably a propylene-α-olefin copolymer, more preferably a propylene-1-butene copolymer because the effects of the present invention can be more effectively expressed.

The content of the Ziegler-Natta-based olefin resin in the hot-melt pressure-sensitive adhesive is preferably from 70 wt% to 20 wt%, more preferably from 65 wt% to 25 wt%, still more preferably from 60 wt% to 30 wt%, particularly preferably from 55 wt% to 35 wt% because the effects of the present invention can be more effectively expressed.

The Ziegler-Natta-based olefin resin is also available as a commercial product. Examples of such commercial product include some products in the "Rextac" (trademark) series (e.g., REXTAC RT 2788 and REXTAC RT 2730) manufactured by REXtac, LLC.

The hot-melt pressure-sensitive adhesive of the present invention includes a metallocene-based olefin resin. The metallocene-based olefin resin is an olefin-based resin obtained by polymerization involving using a metallocene catalyst. The number of kinds of the metallocene-based olefin resins may be only one, or may be two or more.

Any appropriate olefin-based resin may be adopted as the metallocene-based olefin resin to the extent that the effects of the present invention are not impaired as long as the olefin-based resin is obtained by polymerization involving using a metallocene catalyst. Preferred examples of such metallocene-based olefin resin include a homopolymer of an α-olefin and a copolymer of two or more kinds of α-olefins. The α-olefin is preferably an α-olefin having 2 to 20 carbon atoms. Examples of such α-olefin include ethylene, propylene, 1-butene, 1-pentene, 1-hexene, 1-heptene, 1-octene, and 4-methyl-1-pentene. The metallocene-based olefin resin is more preferably a metallocene-based olefin resin free of an aromatic structure. The adoption of the metallocene-based olefin resin free of an aromatic structure can provide a hot-melt pressure-sensitive adhesive more sufficiently suppressed in odor. The metallocene-based olefin resin is preferably a propylene-based elastomer because the effects of the present invention can be more effectively expressed.

The content of the metallocene-based olefin resin in the hot-melt pressure-sensitive adhesive is from 8 wt% to 0.1 wt%, preferably from 5 wt% to 1 wt%, most preferably from 5 wt% to 2 wt%. When the content of the metallocene-based olefin resin in the hot-melt pressure-sensitive adhesive is adjusted within the range, the effects of the present invention can be more effectively expressed, and excellent flexibility can be imparted to a stretchable laminate having the hot-melt pressure-sensitive adhesive of the present invention.

The metallocene-based olefin resin is also available as a commercial product. Examples of such commercial product include some products in the "Tafmer" (trademark) series (e.g., Tafmer PN-3560) manufactured by Mitsui Chemicals, Inc., and some products in the "Vistamaxx" (trademark) series (e.g., Vistamaxx 3000, Vistamaxx 6202, Vistamaxx 7010, and Vistamaxx 7050) manufactured by Exxon Mobil Corporation.

The number of kinds of the hydrocarbon-based tackifiers may be only one, or may be two or more.

Any appropriate hydrocarbon-based tackifier may be adopted as the hydrocarbon-based tackifier to the extent that the effects of the present invention are not impaired. Examples of such hydrocarbon-based tackifier include an aliphatic hydrocarbon-based tackifier, an alicyclic hydrocarbon-based tackifier, an aliphatic-alicyclic hydrocarbon-based tackifier, and a hydrogenated hydrocarbon-based tackifier. The hydrocarbon-based tackifier is more preferably a hydrocarbon-based tackifier free of an aromatic structure. The adoption of the hydrocarbon-based tackifier free of an aromatic structure can provide a hot-melt pressure-sensitive adhesive more sufficiently suppressed in odor.

The content of the hydrocarbon-based tackifier in the hot-melt pressure-sensitive adhesive is from 60 wt% to 30 wt%. When the content of the hydrocarbon-based tackifier in the hot-melt pressure-sensitive adhesive is adjusted within the range, the pressure-sensitive adhesive property of the hot-melt pressure-sensitive adhesive of the present invention can be sufficiently expressed.

The number of kinds of the oil components may be only one, or may be two or more.

Any appropriate oil component may be adopted as the oil component to the extent that the effects of the present invention are not impaired. When the hot-melt pressure-sensitive adhesive of the present invention includes the oil component, the viscosity of the hot-melt pressure-sensitive adhesive of the present invention can be appropriately adjusted, and the pressure-sensitive adhesive property of the hot-melt pressure-sensitive adhesive of the present invention can be appropriately adjusted. Such oil component is preferablyaparaffinoil, such as liquid paraffin. Whenthehot-melt pressure-sensitive adhesive of the present invention includes the paraffin oil, a hot-melt pressure-sensitive adhesive more sufficiently suppressed in odor can be provided.

The content of the oil component in the hot-melt pressure-sensitive adhesive is preferably from 40 wt% to 5 wt%, more preferably from 35 wt% to 6 wt%, still more preferably from 30 wt% to 8 wt%, particularly preferably from 25 wt% to 10 wt%. When the content of the oil component in the hot-melt pressure-sensitive adhesive is adjusted within the range, the viscosity of the hot-melt pressure-sensitive adhesive of the present invention can be more appropriately adjusted, and the pressure-sensitive adhesive property of the hot-melt pressure-sensitive adhesive of the present invention can be appropriately adjusted. In addition, when the content of the oil component in the hot-melt pressure-sensitive adhesive is adjusted within the range, a hot-melt pressure-sensitive adhesive even more sufficiently suppressed in odor can be provided.

The hot-melt pressure-sensitive adhesive of the present invention may include any appropriate other component to the extent that the effects of the present invention are not impaired. Examples of such other component include liquid paraffin, an antioxidant, a UV absorber, a light stabilizer, and a fluorescence agent. The number of kinds of such other components may be only one, or may be two or more.

The adhesive strength of the hot-melt pressure-sensitive adhesive of the present invention at 23°C, a peel angle of 90°, and a peel rate of 300 mm/min on a polypropylene spunbonded non-woven fabric having a basis weight of 19 g/m² is 200 g/25 mm or more, preferably 250 g/25 mm or more, more preferably 300 g/25 mm or more, still more preferably 320 g/25 mm or more, particularly preferably 340 g/25 mm or more, most preferably 350 g/25 mm or more. Ideally, an upper limit value for the adhesive strength is desirably as high as possible. In reality, however, the upper limit value is, for example, preferably 1,000 g/25 mm, more preferably 900 g/25 mm, still more preferably 800 g/25 mm, particularly preferably 700 g/25 mm. When the adhesive strength of the hot-melt pressure-sensitive adhesive of the present invention at 23°C, a peel angle of 90°, and a peel rate of 3 00 mm/min on the polypropylene spunbonded non-woven fabric having a basis weight of 19 g/m² is adjusted within the range, the adhesive property of the hot-melt pressure-sensitive adhesive of the present invention on the non-woven fabric can be sufficiently expressed, and hence the pressure-sensitive adhesive can be suitably used in, for example, the bonding of the non-woven fabric layer of a stretchable laminate serving as a member for an article such as a sanitary article, for example, a diaper or a mask. Details about the measurement of the adhesive strength at 23°C, a peel angle of 90°, and a peel rate of 300 mm/min on the polypropylene spunbonded non-woven fabric having a basis weight of 19 g/m² are described later.

The adhesive strength of the hot-melt pressure-sensitive adhesive of the present invention at 23°C, a peel angle of 90°, and a peel rate of 300 mm/min on a stainless-steel plate is 700 g/25 mm or more, preferably 750 g/25 mm or more, more preferably 800 g/25 mm or more, still more preferably 850 g/25 mm or more, yet still more preferably 900 g/25 mmormore, particularly preferably 950 g/25 mm or more, most preferably 1,000 g/25 mm or more. Ideally, an upper limit value for the adhesive strength is desirably as high as possible. In reality, however, the upper limit value is, for example, preferably 1,500 g/25 mm, more preferably 1,400 g/25 mm, still more preferably 1,300 g/25 mm, particularly preferably 1,200 g/25 mm. When the adhesive strength of the hot-melt pressure-sensitive adhesive of the present invention at 23°C, a peel angle of 90°, and a peel rate of 300 mm/min on the stainless - steel plate is adjusted within the range, the adhesive property of the hot-melt pressure-sensitive adhesive of the present invention on the non-woven fabric can be sufficiently expressed, and hence the pressure-sensitive adhesive can be suitably used in, for example, the bonding of the non-woven fabric layer of a stretchable laminate serving as a member for an article such as a sanitary article, for example, a diaper or a mask. Details about the measurement of the adhesive strength at 23°C, a peel angle of 90°, and a peel rate of 300 mm/min on the stainless-steel plate are described later.

The melt viscosity of the hot-melt pressure-sensitive adhesive of the present invention at 160°C is 10,000 mPa·s or less, preferably 9,000 mPa·s or less, more preferably 8,000 mPa·s or less, still more preferably 7,000 mPa·s or less, particularly preferably 6,000 mPa·s or less, most preferably 5,000 mPa·s or less. In reality, a lower limit value for the melt viscosity at 160°C is preferably 2,000 mPa·s. When the melt viscosity of the hot-melt pressure-sensitive adhesive of the present invention at 160°C is adjusted within the range, the hot-melt pressure-sensitive adhesive of the present invention can be evenly applied in a low temperature region near 160°C.

The melt viscosity of the hot-melt pressure-sensitive adhesive of the present invention at 140°C is 15,000 mPa·s or less, preferably 12,000 mPa·s or less, more preferably 11,000 mPa·s or less, still more preferably 10,000 mPa·s or less, particularly preferably 9,500 mPa·s or less, most preferably 9,000 mPa·s or less. In reality, a lower limit value for the melt viscosity at 140°C is preferably 2,000 mPa·s. When the melt viscosity of the hot-melt pressure-sensitive adhesive of the present invention at 140°C is adjusted within the range, the hot-melt pressure-sensitive adhesive of the present invention can be evenly applied in a low temperature region near 140°C.

The softening point temperature of the hot-melt pressure-sensitive adhesive of the present invention is 100°C or less, preferably 98°C or less, more preferably 96°C or less, still more preferably 95°C or less, particularly preferably 94°C or less, most preferably 93°C or less. In reality, a lower limit value for the softening point temperature of the hot-melt pressure-sensitive adhesive of the present invention is preferably 80°C. When the softening point temperature of the hot-melt pressure-sensitive adhesive of the present invention is adjusted within the range, the hot-melt pressure-sensitive adhesive of the present invention can be easily melted in, for example, a melting tank, and hence a stretchable laminate using the hot-melt pressure-sensitive adhesive of the present invention can be continuously produced.

### <<<<Stretchable Laminate>>>>

A stretchable laminate of the present invention is a stretchable laminate including an elastomer layer and a non-woven fabric layer arranged on at least one side of the elastomer layer. The stretchable laminate of the present invention may include any appropriate other layer to the extent that the effects of the present invention are not impaired as long as the stretchable laminate includes the non-woven fabric layer on at least one side of the elastomer layer. The number of such any appropriate other layers may be only one, or may be two or more.

The stretchable laminate of the present invention has the hot-melt pressure-sensitive adhesive of the present invention between the elastomer layer and the non-woven fabric layer.

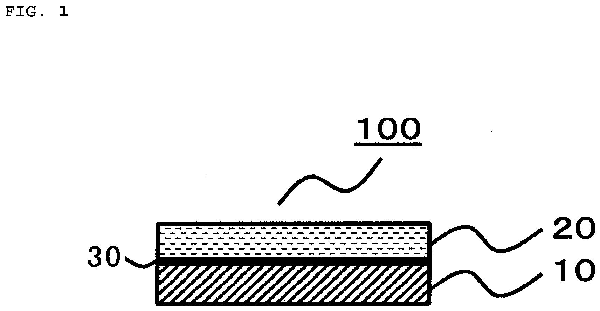

FIG. **1** is a schematic sectional view of a stretchable laminate according to a preferred embodiment of the present invention. A stretchable laminate **100** illustrated in FIG. **1** includes an elastomer layer **10** and a non-woven fabric layer **20** arranged only on one side of the elastomer layer **10.** The stretchable laminate **100** illustrated in FIG. **1** has a hot-melt pressure-sensitive adhesive **30** of the present invention between the elastomer layer **10** and the non-woven fabric layer **20.** In FIG. **1****,** the hot-melt pressure-sensitive adhesive **30** of the present invention is drawn so as to be present in the entirety of a space between the elastomer layer **10** and the non-woven fabric layer **20.** However, the hot-melt pressure-sensitive adhesive **30** of the present invention may be present in part of the space between the elastomer layer **10** and the non-woven fabric layer **20.**

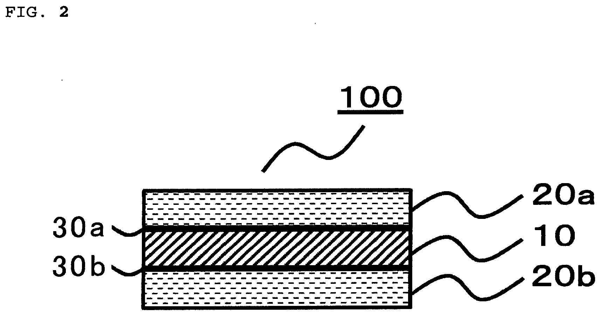

FIG. **2** is a schematic sectional view of another stretchable laminate according to a preferred embodiment of the present invention. A stretchable laminate **100** illustrated in FIG. **2** includes an elastomer layer **10,** a non-woven fabric layer **20a** arranged on one side of the elastomer layer **10,** and a non-woven fabric layer **20b** arranged on the elastomer layer **10** on an opposite side to the non-woven fabric layer **20a.** The stretchable laminate **100** illustrated in FIG. **2** has a hot-melt pressure-sensitive adhesive **30a** of the present invention between the elastomer layer **10** and the non-woven fabric layer **20a,** and has a hot-melt pressure-sensitive adhesive **30b** of the present invention between the elastomer layer **10** and the non-woven fabric layer **20b.** In FIG. **2****,** the hot-melt pressure-sensitive adhesive **30a** of the present invention is drawn so as to be present in the entirety of a space between the elastomer layer 10 and the non-woven fabric layer **20a.** However, the hot-melt pressure-sensitive adhesive **30a** of the present invention may be present in part of the space between the elastomer layer **10** and the non-woven fabric layer **20a.** In addition, similarly, in FIG. **2****,** the hot-melt pressure-sensitive adhesive **30b** of the present invention is drawn so as to be present in the entirety of a space between the elastomer layer **10** and the non-woven fabric layer **20b.** However, the hot-melt pressure-sensitive adhesive **30b** of the present invention may be present in part of the space between the elastomer layer **10** and the non-woven fabric layer **20b.**

The thickness of the stretchable laminate of the present invention varies depending on the thickness of the elastomer layer or the thickness of the non-woven fabric layer, and is preferably from 1.0 mm to 0.1 mm, more preferably from 0.8 mm to 0.15 mm, still more preferably from 0.6 mm to 0.15 mm, particularly preferably from 0.5 mm to 0.2 mm, most preferably from 0.45 mm to 0.2 mm. When the thickness of the stretchable laminate of the present invention falls within such range, the laminate can be easily used as a member used in an article such as a sanitary article, for example, a diaper or a mask.

### <<Elastomer Layer>>

Any appropriate elastomer layer may be adopted as the elastomer layer to the extent that the effects of the present invention are not impaired. Examples of an elastomer resin serving as a main component of such elastomer layer include an olefin-based elastomer, a styrene-based elastomer, a vinyl chloride-based elastomer, a urethane-based elastomer, an ester-based elastomer, and an amide-based elastomer.

The content of the elastomer resin serving as the main component in the elastomer layer is preferably from 50 wt% to 100 wt%, more preferably from 70 wt% to 100 wt%, still more preferably from 90 wt% to 100 wt%, particularly preferably from 95 wt% to 100 wt%, most preferably from 98 wt% to 100 wt%. When the content of the elastomer resin serving as the main component in the elastomer layer falls within the range, the elastomer layer can express a sufficient elastomer characteristic.

The number of the elastomer layers may be one, or may be two or more. When the elastomer layer has a three-layer structure, the three-layer structure is preferably, for example, such a three-layer structure that a layer in which two or more kinds of elastomers are blended is used as an intermediate layer, and layers each containing one of elastomers of the same kinds as the elastomers in the intermediate layer are used as both surface layers.

In the present invention, the elastomer resin serving as the main component in the elastomer layer is preferably an olefin-based elastomer. When the olefin-based elastomer is adopted as the elastomer resin, heat stability is improved as compared to any other elastomer resin (e.g., a styrene-based elastomer), and hence, for example, heat decomposition at the time of the formation of the resin into a film in the production of the stretchable laminate of the present invention can be suppressed. In addition, when the olefin-based elastomer is adopted as the elastomer resin, storage stability is improved as compared to any other elastomer resin (e.g., a styrene-based elastomer), and hence the fluctuation of values for physical properties during the storage of the stretchable laminate of the present invention can be suppressed.

In the present invention, when the olefin-based elastomer is adopted as the elastomer resin, steps in the production of the elastomer layer can be simplified, and hence processing cost can be suppressed. This is because of, for example, the following reason. When any other elastomer resin (e.g., a styrene-based elastomer) is adopted as the elastomer resin, several kinds of styrene-based elastomers need to be blended for controlling values for physical properties. To this end, a master batch needs to be produced. When the olefin-based elastomer is adopted as the elastomer resin, extrusion molding can be performed by using fewer kinds of resins in the production of the elastomer layer, and hence the need for the production of the master batch can be eliminated.

In the present invention, when the olefin-based elastomer is adopted as the elastomer resin, the olefin-based elastomer may be only one kind of elastomer or a blend of two or more kinds of elastomers.

Examples of the olef in-based elastomer include an olefin block copolymer, an olefin random copolymer, an ethylene copolymer, a propylene copolymer, an ethylene olef in block copolymer, a propylene olefin block copolymer, an ethylene olefin random copolymer, a propylene olefin random copolymer, an ethylene propylene random copolymer, an ethylene (1-butene) random copolymer, an ethylene (1-pentene) olefin block copolymer, an ethylene (1-hexene) random copolymer, an ethylene (1-heptene) olefin block copolymer, an ethylene (1-octene) olefin block copolymer, an ethylene (1-nonene) olefin block copolymer, an ethylene (1-decene) olefin block copolymer, a propylene ethylene olefin block copolymer, an ethylene (a-olefin) copolymer, an ethylene (a-olefin) random copolymer, an ethylene (α-olefin) block copolymer, and combinations thereof.

The olef in-based elastomer that may be adopted as the elastomer resin in the present invention has a density of preferably from 0.890 g/cm³ to 0.830 g/cm³, more preferably from 0.888 g/cm³ to 0.835 g/cm³, still more preferably from 0.886 g/cm³ to 0.835 g/cm³, particularly preferably from 0.885 g/cm³ to 0.840 g/cm³, most preferably from 0.885 g/cm³ to 0.845 g/cm³. When the olefin-based elastomer whose density falls within the range is adopted, a stretchable laminate having more excellent fittability can be provided. In addition, the heat stability is further improved as compared to any other elastomer resin (e.g., a styrene-based elastomer), and hence, for example, the heat decomposition at the time of the formation of the resin into a film in the production of the stretchable laminate of the present invention can be further suppressed. In addition, the storage stability is further improved as compared to any other elastomer resin (e.g., a styrene-based elastomer), and hence the fluctuation of values for physical properties during the storage of the stretchable laminate of the present invention can be further suppressed. Further, the steps in the production of the elastomer layer can be further simplified, and hence the processing cost can be further suppressed.

The olefin-based elastomer that maybe adopted as the elastomer resin in the present invention has a MFR at 230°C and 2.16 kgf of preferably from 1.0 g/10 min to 25.0 g/10 min, more preferably from 2.0 g/10 min to 23.0 g/10 min, still more preferably from 2.0 g/10 min to 21.0 g/10 min, particularly preferably from 2.0 g/10 min to 20.0 g/10 min, most preferably from 2.0 g/10 min to 19.0 g/10 min. When the olefin-based elastomer whose MFR falls within the range is adopted, a stretchable laminate having more excellent fittability can be provided. In addition, the heat stability is further improved as compared to any other elastomer resin (e.g., a styrene-based elastomer), and hence, for example, the heat decomposition at the time of the formation of the resin into a film in the production of the stretchable laminate of the present invention can be further suppressed. In addition, the storage stability is further improved as compared to any other elastomer resin (e.g., a styrene-based elastomer), and hence the fluctuation of values for physical properties during the storage of the stretchable laminate of the present invention can be further suppressed. Further, the steps in the production of the elastomer layer can be further simplified, and hence the processing cost can be further suppressed.

The olefin-based elastomer that maybe adopted as the elastomer resin in the present invention is specifically preferably an α-olefin-based elastomer, that is, a copolymer of two or more kinds of α-olefins having an elastomer characteristic. Of such α-olefin-based elastomers, at least one kind selected from an ethylene-based elastomer, a propylene-based elastomer, and a 1-butene-based elastomer is more preferred. When such α-olefin-based elastomer is adopted as the olefin-based elastomer, a stretchable laminate having more excellent fittability can be provided. In addition, the heat stability is further improved as compared to any other elastomer resin (e.g., a styrene-based elastomer), and hence, for example, the heat decomposition at the time of the formation of the resin into a film in the production of the stretchable laminate of the present invention can be further suppressed. In addition, the storage stability is further improved as compared to any other elastomer resin (e.g., a styrene-based elastomer), and hence the fluctuation of values for physical properties during the storage of the stretchable laminate of the present invention can be further suppressed. Further, the steps in the production of the elastomer layer can be further simplified, and hence the processing cost can be further suppressed.

Of the α-olefin-based elastomers that may be adopted as the elastomer resin in the present invention, a propylene-based elastomer is particularly preferred. When the propylene-based elastomer is adopted as the olefin-based elastomer, a stretchable laminate having extremely excellent fittability can be provided. In addition, the heat stability is still further improved as compared to any other elastomer resin (e.g., a styrene-based elastomer), and hence, for example, the heat decomposition at the time of the formation of the resin into a film in the production of the stretchable laminate of the present invention can be still further suppressed. In addition, the storage stability is still further improved as compared to any other elastomer resin (e.g., a styrene-based elastomer), and hence the fluctuation of values for physical properties during the storage of the stretchable laminate of the present invention can be still further suppressed. Further, the steps in the production of the elastomer layer can be still further simplified, and hence the processing cost can be still further suppressed.

Such α-olefin-based elastomer as described above is also available as a commercial product. Examples of such commercial product include some products in the "Tafmer" (trademark) series (e.g., Tafmer PN-3560) manufactured by Mitsui Chemicals, Inc., and some products in the "Vistamaxx" (trademark) series (e.g. , Vistamaxx 3000, Vistamaxx 6202, and Vistamaxx 7010) manufactured by Exxon Mobil Corporation.

The α-olefin-based elastomer that may be adopted as the elastomer resin in the present invention is preferably produced by using a metallocene catalyst. When the α-olefin-based elastomer produced by using a metallocene catalyst is adopted, a stretchable laminate having extremely excellent fittability can be provided. In addition, the heat stability is still further improved as compared to any other elastomer resin (e.g., a styrene-based elastomer), and hence, for example, the heat decomposition at the time of the formation of the resin into a film in the production of the stretchable laminate of the present invention can be still further suppressed. In addition, the storage stability is still further improved as compared to any other elastomer resin (e.g., a styrene-based elastomer), and hence the fluctuation of values for physical properties during the storage of the stretchable laminate of the present invention can be still further suppressed. Further, the steps in the production of the elastomer layer can be still further simplified, and hence the processing cost can be still further suppressed.

The elastomer layer may contain any appropriate other component as long as the effects of the present invention are not impaired. Examples of such other component include any other polymer, a tackifier, a plasticizer, an antidegradant, a pigment, a dye, an antioxidant, an antistatic agent, a lubricant, a blowing agent, a heat stabilizer, a light stabilizer, an inorganic filler, and an organic filler. The number of kinds of those components may be only one, or may be two or more. The content of the other component in the elastomer layer is preferably 10 wt% or less, more preferably 7 wt% or less, still more preferably 5 wt% or less, particularly preferably 2 wt% or less, most preferably 1 wt% or less.

The thickness of the elastomer layer is preferably from 200 µm to 20 µm, more preferably from 160 µm to 30 µm, still more preferably from 140 µm to 30 µm, particularly preferably from 120 µm to 30 µm, most preferably from 100 µm to 30 µm. When the thickness of the elastomer layer falls within such range, a stretchable laminate having more excellent fittability can be provided.

### <<Non-woven Fabric Layer>>

Any appropriate non-woven fabric layer may be adopted as the non-woven fabric layer as long as the effects of the present invention are not impaired. The number of kinds of non-woven fabrics forming the non-woven fabric layer may be only one, or may be two or more.

Examples of the non-woven fabric forming the non-woven fabric layer include a spunbonded non-woven web, a fluffy non-woven fabric (such as a non-woven fabric obtained by a thermal bonding method, a bonding joining method, or a spunlace method), a meltblown non-woven web, a spunlace non-woven web, a spunbonded meltblown spunbonded non-woven web, a spunbonded meltblown meltblown spunbonded non-woven web, an unjoined non-woven web, an electrospun non-woven web, a flashspun non-woven web (such as TYVEK™ from DuPont), and a carded non-woven fabric.

For example, the non-woven fabric forming the non-woven fabric layer may contain fibers of polypropylene, polyethylene, polyester, polyamide, polyurethane, an elastomer, rayon, cellulose, acrylic, a copolymer thereof, or a blend thereof, or a mixture thereof, or any other polyolefin.

The non-woven fabric forming the non-woven fabric layer may contain fibers as a homogeneous structural body, or may contain a bicomponent structural body, such as a sheath/core structure, a side-by-side structure, a sea-island structure, and any other bicomponent structure. Detailed descriptions of the non-woven fabric may be found in, for example, "Nonwoven Fabric Primer and Reference Sampler," E.A. Vaughn, Association of the Nonwoven Fabrics Industry, third edition (1992).

The basis weight of the non-woven fabric forming the non-woven fabric layer is preferably 150 gsm or less, more preferably 100 gsm or less, still more preferably 50 gsm or less, particularly preferably from 10 gsm to 30 gsm.

### <<Hot-melt Pressure-sensitive Adhesive>>

The stretchable laminate of the present invention has the hot-melt pressure-sensitive adhesive of the present invention between the elastomer layer and the non-woven fabric layer. The use of the hot-melt pressure-sensitive adhesive of the present invention reduces the need for the addition of the tackifier as a component for the elastomer layer, for example, improves the extrusion stability of the layer, and hence can suppress a problem in that the tackifier adheres to a forming roll and can suppress a problem in that a production line is contaminated by, for example, a volatile component resulting from the tackifier. Further, the hot-melt pressure-sensitive adhesive of the present invention can sufficiently suppress the odor of the stretchable laminate of the present invention because the hot-melt pressure-sensitive adhesive is sufficiently suppressed in odor.

When the hot-melt pressure-sensitive adhesive of the present invention is used in the bonding of the elastomer layer and the non-woven fabric layer, the hot-melt pressure-sensitive adhesive may be entirely present on the non-woven fabric layer, or may be partially present on the non-woven fabric layer. When the hot-melt pressure-sensitive adhesive of the present invention is partially present on the non-woven fabric layer, the hot-melt pressure-sensitive adhesive of the present invention is preferably present so as to include at least an end portion of the non-woven fabric layer.

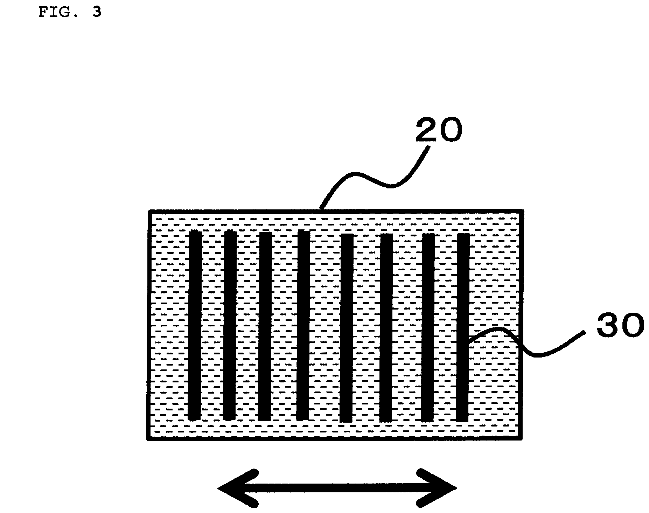

When the hot-melt pressure-sensitive adhesive of the present invention is used in the bonding of the elastomer layer and the non-woven fabric layer, the hot-melt pressure-sensitive adhesive may be present, for example, as follows: the hot-melt pressure-sensitive adhesive 30 of the present invention may be present on the non-woven fabric layer 20 in a stripe manner in the flow direction of a production line as illustrated in FIG. 3, or may be present in a dot manner. When the hot-melt pressure-sensitive adhesive of the present invention is present in a stripe manner, a site where the hot-melt pressure-sensitive adhesive of the present invention is present and a site where the pressure-sensitive adhesive is not present are formed in a stripe manner, and hence the stretchability of the stretchable laminate may be further improved particularly in a direction perpendicular to the stripe (direction indicated by the arrow of FIG. 3).

### <<Production of Stretchable Laminate of the Present Invention>>

At the time of the production of the stretchable laminate of the present invention, the elastomer layer and the non-woven fabric layer need to be bonded to each other because the elastomer layer and the non-woven fabric layer are directly laminated (e.g., FIG. 1 or 2). Examples of such bonding method include (1) a method involving laminating the elastomer layer formed by extrusion from the T-die of an extrusion machine and the non-woven fabric layer separately drawn from a roll body, (2) a method involving subjecting the elastomer layer and the non-woven fabric layer to simultaneous extrusion lamination, (3) a method involving bonding the elastomer layer and the non-woven fabric layer, which have each been separately prepared, with an adhesive, (4) a method involving forming the non-woven fabric layer on the elastomer layer, which has been formed by any appropriate method, by a meltblown method or the like, and (5) a method involving subjecting the elastomer layer and the non-woven fabric layer to thermal lamination or ultrasonic welding.

At the time of the production of the stretchable laminate of the present invention, the hot-melt pressure-sensitive adhesive of the present invention is used in the bonding of the elastomer layer and the non-woven fabric layer. For example, when the method (1) is applied, the top of the non-woven fabric layer separately drawn from the roll body only needs to be coated with the hot-melt pressure-sensitive adhesive of the present invention before the layer is subjected to the lamination with the elastomer layer.

The stretchable laminate of the present invention may be subjected to treatments referred to as pre-stretching treatment and activation treatment after the lamination of the elastomer layer and the non-woven fabric layer. Specifically, stretching treatment may be performed in a width direction of the stretchable laminate, or treatment in which a fiber structure of a part of the region of the non-woven fabric layer is mechanically broken may be performed. When such treatments are performed, the stretchable laminate can be stretched by a smaller force.

### <<Application of Stretchable Laminate of the Present Invention>>

The stretchable laminate of the present invention can be used in any appropriate article in which the effects of the present invention can be effectively utilized. That is, an article of the present invention includes the stretchable laminate of the present invention. A typical example of such article is a sanitary article. Examples of such sanitary article include a diaper (in particular, an ear portion of a disposable diaper), a supporter, and a mask.

### Examples

The present invention is hereinafter specifically described by way of Examples. However, the present invention is by no means limited to these Examples. Test and evaluation methods in Examples and the like are as described below. In addition, "part(s)" means "part(s) by weight" and "%" means "wt%" unless otherwise stated.

### <Measurement of Adhesive Strength on Non-woven Fabric>

A sheet obtained by applying a hot-melt pressure-sensitive adhesive to an OPP film (thickness=35 µm) at 15 g/m² was cut so as to have a width of 25 mm, and was pressure-bonded onto a spunbonded non-woven fabric totally made of polypropylene for a diaper back sheet (19 g/m²) (hydrophobic type) by reciprocating a load of 1 kg twice. After the pressure bonding, the resultant was left at rest for 10 minutes at room temperature (23°C), and the adhesive strength of the pressure-sensitive adhesive was measured at a peel angle of 90° and a peel rate of 300 mm/min.

### <Measurement of Adhesive Strength on SUS>

A sheet obtained by applying a hot-melt pressure-sensitive adhesive to an OPP film (thickness=35 µm) at 15 g/m² was cut so as to have a width of 25 mm, and was pressure-bonded onto a SUS plate (304BA plate) by reciprocating a load of 1 kg twice. After the pressure bonding, the resultant was left at rest for 10 minutes at room temperature (23°C), and the adhesive strength of the pressure-sensitive adhesive was measured at a peel angle of 90° and a peel rate of 300 mm/min.

### <Measurement of Melt Viscosity at 160°C>

The melt viscosity of a hot-melt pressure-sensitive adhesive was measured in Brookfield Cap 2000+ Rotational Viscometer (Brookfield) through the use of a spindle having a number of revolutions of 0.6. A measurement temperature was gradually reduced from 180°C, and the viscosity at 160°C was measured.

### <Measurement of Melt Viscosity at 140°C>

The melt viscosity of a hot-melt pressure-sensitive adhesive was measured in Brookfield Cap 2000+ Rotational Viscometer (Brookfield) through the use of a spindle having a number of revolutions of 0.6. A measurement temperature was gradually reduced from 180°C, and the viscosity at 140°C was measured.

### <Measurement of Softening Point Temperature>

The temperature of a hot-melt pressure-sensitive adhesive was increased with Softening Point Ring & Ball Tester "HRB754" manufactured by Herzog at 5°C/min, and the temperature at which a ball fell was defined as its softening point temperature.

### <Odor Analysis>

1.5 Grams of a hot-melt pressure-sensitive adhesive was used as a sample. The sample was loaded into a glass chamber whose cleanliness had been determined, and its temperature was increased from room temperature (23°C) to 80°C in a heating oven while a high-purity nitrogen gas was flowed at 50 mL/min. After that, the sample was heated for 180 minutes, and a gas component produced from the sample was collected in a canister. The gas component was pressurized by a factor of 2. After the pressurization, a substance in the gas component in the vacuum chamber was identified by microscale purge & trap gas chromatography-mass spectrometry (MPT-GC/MS). At this time, a case in which a gas responsible for an odor, i.e., (1) toluene, (2) styrene, or (3) 1-methyl-5-(1-methylethenyl)cyclohexene was detected was evaluated as ×, and a case in which no gas responsible for an odor was detected was evaluated as o. In this analysis, odor detection was simultaneously performed, and a case in which a strong odor was felt at the time of the resultant peak of any one of the gases (1) to (3) was evaluated as ××.

Used equipment and the like are as described below.
MPT: Model ENT-7100A manufactured by EnTech
GC/MS: Model GC 6890N+MSD 5975B manufactured by Agilent Technologies
Library: WILEY/NIH 736000 substance

### <Evaluation for Flexibility>

A hot-melt pressure-sensitive adhesive was applied to the entire surface of a PP spun lace non-woven fabric (35 g/spm) in an application amount of 3 g/spm. After the application, the non-woven fabric with a hot-melt pressure-sensitive adhesive was stored for 1 day at room temperature (23°C), and was then pulled in its width direction (CD direction) . At that time, a case in which the non-woven fabric was not stretched at all was evaluated as ×, a case in which the non-woven fabric showed a remarkable reduction in flexibility was evaluated as Δ, and a case in which the non-woven fabric did not show a remarkable reduction in flexibility was evaluated as o.

### [Reference Example 1]

667 Parts of a propylene-1-butene copolymer (manufactured by REXtac, LLC, product name: REXTAC RT 2788) serving as a Ziegler-Natta-based olefin resin, 520 parts of a hydrocarbon-based tackifier (manufactured by Kolon Industries, Inc., product name: SUKOREZ SU-100 S), 100 parts of liquid paraffin (manufactured by Petro yag, product name: White Oil Pharma Oyster 259), and 13 parts of an antioxidant (manufactured by BASF, product name: Irganox 1010) were blended to provide a hot-melt pressure-sensitive adhesive (1) .

The results are shown in Table 1.

### [Example 2]

517 Parts of a propylene-1-butene copolymer (manufactured by REXtac, LLC, product name: REXTAC RT 2788) serving as a Ziegler-Natta-based olefin resin, 100 parts of a metallocene-based olefin-based resin (manufactured by ExxonMobil Corporation, product name: Vistamaxx 6202), 520 parts of a hydrocarbon-based tackifier (manufactured by Exxon Mobil Corporation, product name: Escorez 5400), 150 parts of liquid paraffin (manufactured by Petro yag, product name: White Oil Pharma Oyster 259), and 13 parts of an antioxidant (manufactured by BASF, product name: Irganox 1010) were blended to provide a hot-melt pressure-sensitive adhesive (2).

The results are shown in Table 1.

### [Example 3]

517 Parts of a propylene-1-butene copolymer (manufactured by REXtac, LLC, product name: REXTAC RT 2730) serving as a Ziegler-Natta-based olefin resin, 100 parts of a metallocene-based olefin-based resin (manufactured by ExxonMobil Corporation, product name: Vistamaxx 7050), 520 parts of a hydrocarbon-based tackifier (manufactured by Exxon Mobil Corporation, product name: Escorez 5400), 150 parts of liquid paraffin (manufactured by Petro yag, product name: White Oil Pharma Oyster 259), and 13 parts of an antioxidant (manufactured by BASF, product name: Irganox 1010) were blended to provide a hot-melt pressure-sensitive adhesive (3).

The results are shown in Table 1.

### [Example 4]

517 Parts of a propylene-1-butene copolymer (manufactured by REXtac, LLC, product name: REXTAC RT 2788) serving as a Ziegler-Natta-based olefin resin, 100 parts of a metallocene-based olefin-based resin (manufacturedby ExxonMobil Corporation, product name: Vistamaxx 7050), 520 parts of a hydrocarbon-based tackifier (manufactured by Exxon Mobil Corporation, product name: Escorez 5400), 150 parts of liquid paraffin (manufactured by Petro yag, product name: White Oil Pharma Oyster 259), and 13 parts of an antioxidant (manufactured by BASF, product name: Irganox 1010) were blended to provide a hot-melt pressure-sensitive adhesive (4).

The results are shown in Table 1.

### [Example 5]

562 Parts of a propylene-1-butene copolymer (manufactured by REXtac, LLC, product name: REXTAC RT 2788) serving as a Ziegler-Natta-based olefin resin, 55 parts of a metallocene-based olefin-based resin (manufactured by Exxon Mobil Corporation, product name: Vistamaxx 7050), 520 parts of a hydrocarbon-based tackifier (manufactured by Kolon Industries, Inc., product name: SUKOREZ SU-100 S), 150 parts of liquid paraffin (manufactured by Petro yag, product name: White Oil Pharma Oyster 259), and 13 parts of an antioxidant (manufactured by BASF, product name: Irganox 1010) were blended to provide a hot-melt pressure-sensitive adhesive (5).

The results are shown in Table 1.

### [Reference Example 6]

400 Parts of a propylene-1-butene copolymer (manufactured by REXtac, LLC, product name: REXTAC RT 2788) serving as a Ziegler-Natta-based olefin resin, 267 parts of a propylene-1-butene copolymer (manufactured by REXtac, LLC, product name: REXTAC RT 2730) serving as a Ziegler-Natta-based olefin resin, 520 parts of a hydrocarbon-based tackifier (manufactured by Kolon Industries, Inc., product name: SUKOREZ SU-100 S), 100 parts of liquid paraffin (manufactured by Petro yag, product name: White Oil Pharma Oyster 259), and 13 parts of an antioxidant (manufactured by BASF, product name: Irganox 1010) were blended to provide a hot-melt pressure-sensitive adhesive (6).

The results are shown in Table 1.

### [Example 7]

300 Parts of a propylene-1-butene copolymer (manufactured by REXtac, LLC, product name: REXTAC RT 2788) serving as a Ziegler-Natta-based olefin resin, 267 parts of a propylene-1-butene copolymer (manufactured by REXtac, LLC, product name: REXTAC RT 2730) serving as a Ziegler-Natta-based olefin resin, 100 parts of a metallocene-based olefin-based resin (manufactured by Exxon Mobil Corporation, product name: Vistamaxx 7050), 520 parts of a hydrocarbon-based tackifier (manufactured by Kolon Industries, Inc., product name: SUKOREZ SU-100 S), 100 parts of liquid paraffin (manufactured by Petro yag, product name: White Oil Pharma Oyster 259), and 13 parts of an antioxidant (manufactured by BASF, product name: Irganox 1010) were blended to provide a hot-melt pressure-sensitive adhesive (7).

The results are shown in Table 1.

### [Example 8]

300 Parts of a propylene-1-butene copolymer (manufactured by REXtac, LLC, product name: REXTAC RT 2788) serving as a Ziegler-Natta-based olefin resin, 217 parts of a propylene-1-butene copolymer (manufactured by REXtac, LLC, product name: REXTAC RT 2730) serving as a Ziegler-Natta-based olefin resin, 100 parts of a metallocene-based olef in-based resin (manufactured by Exxon Mobil Corporation, product name: Vistamaxx 7050), 520 parts of a hydrocarbon-based tackifier (manufactured by Kolon Industries, Inc., product name: SUKOREZ SU-100 S), 150 parts of liquid paraffin (manufactured by Petro yag, product name: White Oil Pharma Oyster 259), and 13 parts of an antioxidant (manufactured by BASF, product name: Irganox 1010) were blended to provide a hot-melt pressure-sensitive adhesive (8).

The results are shown in Table 1.

### [Comparative Example 1]

200 Parts of a propylene-1-butene copolymer (manufactured by REXtac, LLC, product name: REXTAC RT 2788) serving as a Ziegler-Natta-based olefin resin, 467 parts of a metallocene-based olefin-based resin (manufactured by Exxon Mobil Corporation, product name: Vistamaxx 6202), 520 parts of a hydrocarbon-based tackifier (manufactured by Exxon Mobil Corporation, product name: Escorez 5400), 100 parts of liquid paraffin (manufactured by Petro yag, product name: White Oil Pharma Oyster 259), and 13 parts of an antioxidant (manufactured by BASF, product name: Irganox 1010) were blended to provide a hot-melt pressure-sensitive adhesive (C1).

The results are shown in Table 2.

### [Comparative Example 2]

467 Parts of a propylene-1-butene copolymer (manufactured by REXtac, LLC, product name: REXTAC RT 2788) serving as a Ziegler-Natta-based olefin resin, 200 parts of a metallocene-based olefin-based resin (manufactured by ExxonMobil Corporation, product name: Vistamaxx 6202), 520 parts of a hydrocarbon-based tackifier (manufactured by Exxon Mobil Corporation, product name: Escorez 5400), and 100 parts of liquid paraffin (manufactured by Petro yag, product name: White Oil Pharma Oyster 259) were blended to provide a hot-melt pressure-sensitive adhesive (C2).

The results are shown in Table 2.

### [Comparative Example 3]

150 Parts of a propylene-1-butene copolymer (manufactured by REXtac, LLC, product name: REXTAC RT 2788) serving as a Ziegler-Natta-based olefin resin, 213 parts of a SIS-based resin (manufactured by Kraton Polymers, Inc., product name: Kraton D1165 PT), 310 parts of a hydrocarbon-based tackifier (manufactured by Kolon Industries, Inc., product name: SUKOREZ SU-100 S), 309 parts of a hydrocarbon-based tackifier (manufactured by Eastman Chemical Company, product name: Eastotac H-100W), 84 parts of liquid paraffin (manufactured by Petro yag, product name: White Oil Pharma Oyster 259), and 10 parts of an antioxidant (manufactured by BASF, product name: Irganox 1010) were blended to provide a hot-melt pressure-sensitive adhesive (C3).

The results are shown in Table 2.

### [Comparative Example 4]

150 Parts of a propylene-1-butene copolymer (manufactured by REXtac, LLC, product name: REXTAC RT 2788) serving as a Ziegler-Natta-based olefin resin, 213 parts of a SIS-based resin (manufactured by Kraton Polymers, Inc., product name: Kraton D1165 PT), 310 parts of a hydrocarbon-based tackifier (manufactured by Kolon Industries, Inc., product name: SUKOREZ SU-100 S), 309 parts of a hydrocarbon-based tackifier (manufactured by Arakawa Chemical Industries, Ltd., product name: Arkon P-125), 84 parts of liquid paraffin (manufactured by Petro yag, product name: White Oil Pharma Oyster 259), and 10 parts of an antioxidant (manufactured by BASF, product name: Irganox 1010) were blended to provide a hot-melt pressure-sensitive adhesive (C4).

The results are shown in Table 2.

### [Comparative Example 5]

213 Parts of a SIS-based resin (manufactured by Kraton Polymers, Inc. , product name: KratonD1165 PT), 619 parts of a hydrocarbon-based tackifier (manufactured by Kolon Industries, Inc., product name: SUKOREZ SU-100 S), 84 parts of liquid paraffin (manufactured by Petro yag, product name: White Oil Pharma Oyster 259), and 10 parts of an antioxidant (manufactured by BASF, product name: Irganox 1010) were blended to provide a hot-melt pressure-sensitive adhesive (C5) .

The results are shown in Table 2.

### [Comparative Example 6]

35 Parts of a propylene-1-butene copolymer (manufactured by REXtac, LLC, product name: REXTAC RT 2788) serving as a Ziegler-Natta-based olefin resin, 35 parts of a propylene-1-butene copolymer (manufactured by REXtac, LLC, product name: REXTAC RT 2730) serving as a Ziegler-Natta-based olefin resin, 120 parts of a SIS-based resin (manufactured by Kraton Polymers, Inc., product name: Kraton D1119), 120 parts of a SBS-based resin (manufactured by Kraton Polymers, Inc., product name: Kraton KD1102 AS), 353 parts of a hydrocarbon-based tackifier (manufactured by Kolon Industries, Inc., product name: SUKOREZ SU-100 S), 353 parts of a hydrocarbon-based tackifier (manufactured by Exxon Mobil Corporation, product name: Escorez 5400), 39 parts of a hydrocarbon-based tackifier (manufactured by Eastman Chemical Company, product name: Regalite R-1010), 265 parts of liquid paraffin (manufactured by Petro yag, product name: White Oil Pharma Oyster 259), and 10 parts of an antioxidant (manufactured by BASF, product name: Irganox 1010) were blended to provide a hot-melt pressure-sensitive adhesive (C6).

The results are shown in Table 2.

### [Comparative Example 7]

35 Parts of a propylene-1-butene copolymer (manufactured by REXtac, LLC, product name: REXTAC RT 2788) serving as a Ziegler-Natta-based olefin resin, 35 parts of a propylene-1-butene copolymer (manufactured by REXtac, LLC, product name: REXTAC RT 2730) serving as a Ziegler-Natta-based olefin resin, 240 parts of a SBS-based resin (manufacturedbyDexco, product name: Vector 8580A), 706 parts of a hydrocarbon-based tackifier (manufactured by Eastman Chemical Company, product name: Eastotac H-100W), 39 parts of a hydrocarbon-based tackifier (manufactured by Eastman Chemical Company, product name: RegaliteR-1010), 265 parts of liquidparaffin (manufactured by Petro yag, product name: White Oil Pharma Oyster 259), and 10 parts of an antioxidant (manufactured by BASF, product name: Irganox 1010) were blended to provide a hot-melt pressure-sensitive adhesive (C7).

The results are shown in Table 2.

### [Comparative Example 8]

35 Parts of a propylene-1-butene copolymer (manufactured by REXtac, LLC, product name: REXTAC RT 2788) serving as a Ziegler-Natta-based olefin resin, 35 parts of a propylene-1-butene copolymer (manufactured by REXtac, LLC, product name: REXTAC RT 2730) serving as a Ziegler-Natta-based olefin resin, 265 parts of a SBS-based resin (manufactured by Kraton Polymers, Inc., product name: Kraton D0243 ET), 631 parts of a hydrocarbon-based tackifier (manufactured by Eastman Chemical Company, product name: Eastotac H-100W), 39 parts of a hydrocarbon-based tackifier (manufactured by Eastman Chemical Company, product name: Regalite R-1010), 315 parts of liquid paraffin (manufactured by Petro yag, product name: White Oil Pharma Oyster 259), and 10 parts of an antioxidant (manufactured by BASF, product name: Irganox 1010) were blended to provide a hot-melt pressure-sensitive adhesive (C8).

The results are shown in Table 2.

**Table 1**

| | | Example (Examples 1 and 6 are Reference Examples) | | | | | | | |
|---|---|---|---|---|---|---|---|---|---|
| | | 1 | 2 | 3 | 4 | 5 | 6 | 7 | 8 |
| REXTAC RT 2788 | Ziegler-Natt a-based olefin resin | 667 | 517 | | 517 | 562 | 400 | 300 | 300 |
| REXTAC RT 2730 | | | | 517 | | | 267 | 267 | 217 |
| Vistamaxx 6202 | Metallocene-based olefin resin | | 100 | | | | | | |
| Vistamaxx 7050 | | | | 100 | 100 | 55 | | 100 | 100 |
| SUKOREZ SU-100 S | Hydrocarbon-based tackifier | 520 | | | | 520 | 520 | 520 | 520 |
| Escorez 5400 | | | 520 | 520 | 520 | | | | |
| White Oil Pharma | Paraffin oil | 100 | 150 | 150 | 150 | 150 | 100 | 100 | 150 |
| Irganox 1010 | Antioxidant | 13 | 13 | 13 | 13 | 13 | 13 | 13 | 13 |
| | | | | | | | | | |
| Adhesive strength on PP non-woven fabric (peeling at 90°) | g/25 mm | 423 | 350 | 356 | 367 | 326 | 246 | 388 | 343 |
| Adhesive strength on SUS plate (peeling at 90°) | g/25 mm | 888 | 766 | 1,176 | 940 | 870 | 1,280 | 1,211 | 1,029 |
| Melt viscosity at 160°C | mPa·s | 4,909 | 8,866 | 4,447 | 7,890 | 5,650 | 4,103 | 7,806 | 6,141 |
| Melt viscosity at 140°C | mPa·s | 9,254 | 14,503 | 8,213 | 14,684 | 10,515 | 7,443 | 14,812 | 11,514 |
| Evaluation test for flexibility | - | Δ | ○ | ○ | ○ | ○ | Δ | ○ | ○ |
| Softening point temperature | °C | 97 | 92.3 | 85.9 | 91.9 | 94.7 | 92.5 | 94.7 | 93.3 |
| Odor analysis toluene | - | ○ | ○ | ○ | ○ | ○ | ○ | ○ | ○ |
| Odor analysis styrene | - | ○ | ○ | ○ | ○ | ○ | ○ | ○ | ○ |
| Odor analysis 1-methyl-5-(1-methy lethenyl)-cyclohexe ne | - | ○ | ○ | ○ | ○ | ○ | ○ | ○ | ○ |

**Table 2**

| | | Comparative Example | | | | | | | |
|---|---|---|---|---|---|---|---|---|---|
| | | 1 | 2 | 3 | 4 | 5 | 6 | 7 | 8 |
| REXTAC RT 2788 | Ziegler-Natt a-based olefin resin | 200 | 467 | 150 | 150 | | 35 | 35 | 35 |
| REXTAC RT 2730 | | | | | | | 35 | 35 | 35 |
| Vistamaxx 6202 | Metallocene-based olefin resin | 467 | 200 | | | | | | |
| Vistamaxx 7050 | | | | | | | | | |
| Kraton D1165 PT | Styrene-base d resin (SIS) | | | 213 | 213 | 213 | | | |
| Kraton D1119 | | | | | | | 120 | | |
| Kraton KD 1102 AS | Styrene-base d resin (SBS) | | | | | | 120 | | |
| Vector 8508 A | | | | | | | | 240 | |
| Kraton D 0243 ET | | | | | | | | | 265 |
| SUKOREZ SU-100 S | Hydrocarbon-based tackifier | | | 310 | 310 | 619 | 353 | | |
| Eastotac H-100W | | | | 309 | | | | 706 | 631 |
| Arkon P-125 | | | | | 309 | | | | |
| Escorez 5400 | | 520 | 520 | | | | 353 | | |
| Regalite R-1010 | | | | | | | 39 | 39 | 39 |
| White Oil Pharma | Paraffin oil | 100 | 100 | 84 | 84 | 84 | 265 | 265 | 315 |
| Irganox 1010 | Antioxidant | 13 | | 10 | 10 | 10 | 10 | 10 | 10 |
| | | | | | | | | | |
| Adhesive strength on PP non-woven fabric (peeling at 90°) | g/25 mm | 350 | Unmeasu rable owing to separat ion failure | 54 | 44 | 70 | 180 | 192 | 124 |
| Adhesive strength on SUS plate (peeling at 90°) | g/25 mm | 766 | | 427 | 423 | 451 | 800 | 1,014 | 1,260 |
| Melt viscosity at 160°C | mPa·s | 13,553 | | 9,551 | 13,617 | 5,045 | 3,600 | 6,438 | 6,915 |
| Melt viscosity at 140°C | mPa·s | 20,552 | | 15,885 | 29, 141 | 14,188 | 6,800 | 11,255 | 11,248 |
| Evaluation test for flexibility | - | ○ | ○ | ○ | ○ | ○ | ○ | ○ | ○ |
| Softening point temperature | °C | 106.4 | | 97.7 | 113.4 | 101.4 | 92 | 88.1 | 94.9 |
| Odor analysis toluene | - | ○ | ○ | × | × | × | × | ×× | × |
| Odor analysis styrene | - | ○ | ○ | ×× | ×× | ×× | × | × | ×× |
| Odor analysis 1-methyl-5-(1-methy lethenyl)-cyclohexe ne | - | ○ | ○ | × | × | × | ×× | ○ | ○ |

### [Reference Example 9]

100 Weight percent of an olefin-based resin (manufactured by Exxon Mobil Corporation, product name: Vistamaxx 6202) was loaded into an A layer in an extrusion machine, and a formulation of 65 wt% of an olefin-based resin (manufactured by Exxon Mobil Corporation, product name: Vistamaxx 6202), 30 wt% of an olefin-based resin (manufactured by Mitsui Chemicals, Inc., product name: Tafmer PN-3560), and 5 wt% of a white pigment (titanium oxide, manufactured by DuPont, product name: Ti-Pure R103) was loaded into a B layer in the extrusion machine to extrude an elastic film (1) having the construction of A layer/B layer/A layer=6.75 µm/31.5 µm/6.75 µm in total of 45 µm.

An elastomer layer (hereinafter sometimes referred to as "elastic film") was formed by extrusion molding by using the resultant elastic film (1) and the hot-melt pressure-sensitive adhesive (1) obtained in Reference Example 1 with an extrusion T-die molding machine for three layers in two types (A layer/B layer/A layer). The extrusion temperatures were as follows: the extrusion temperature of the A layer was 200°C, the extrusion temperature of the B layer was 200°C, and a die temperature was 200°C.

A non-woven fabric (PP carded type, basis weight=24 gsm) was bonded to each of both surfaces of the elastic film extruded from the T-die with a roll. Thus, a stretchable laminate was obtained. At this time, the hot-melt pressure-sensitive adhesive (1) was applied to the side to which the non-woven fabric was bonded so that a 30-millimeter wide portion (A) whose surface had entirely applied thereto the hot-melt pressure-sensitive adhesive (1) (7 g/m²) and a 41-millimeter wide portion (B) in which the hot-melt pressure-sensitive adhesive (1) was applied (15 g/m²) in a stripe manner (width of the pressure-sensitive adhesive: 1 mm, interval: 1 mm) were alternately present.

Further, the 30-millimeter wide portion (A) whose surface had entirely applied thereto the hot-melt pressure-sensitive adhesive (1) (7 g/m²) was cut at its center. Thus, there was obtained a stretchable laminate (1) having, on each of both surfaces of the elastic film, two 15-millimeter wide portions (A1) and (A2) on both ends whose surfaces each had entirely applied thereto the hot-melt pressure-sensitive adhesive (7 g/m²), and the 41-millimeter wide portion (B) present between the portions (A1) and (A2) in which the hot-melt pressure-sensitive adhesive was applied (15 g/m²) in a stripe manner (width of the pressure-sensitive adhesive: 1 mm, interval: 1 mm).

The hot-melt pressure-sensitive adhesive of the present invention can be suitably used in the bonding of the non-woven fabric layer of a stretchable laminate serving as a member for an article such as a sanitary article, for example, a diaper or a mask. The stretchable laminate of the present invention can be used in any appropriate article in which the effects of the present invention can be effectively utilized. That is, the article of the present invention includes the stretchable laminate of the present invention. A typical example of such article is a sanitary article. Examples of such sanitary article include a diaper (in particular, an ear portion of a disposable diaper), a supporter, and a mask.

### Reference Signs List

- 100: stretchable laminate
- 10: elastomer layer
- 20: non-woven fabric layer
- 20a: non-woven fabric layer
- 20b: non-woven fabric layer
- 30: hot-melt pressure-sensitive adhesive
- 30a: hot-melt pressure-sensitive adhesive
- 30b: hot-melt pressure-sensitive adhesive

## Claims

1. A hot-melt pressure-sensitive adhesive, comprising:
a Ziegler-Natta-based olefin resin;
a metallocene-based olefin resin;
a hydrocarbon-based tackifier; and
an oil component,
wherein the hot-melt pressure-sensitive adhesive has:
an adhesive strength at 23°C, a peel angle of 90°, and a peel rate of 300 mm/min on a polypropylene spunbonded non-woven fabric having a basis weight of 19 g/m² of 200 g/25 mm or more;
an adhesive strength at 23°C, a peel angle of 90°, and a peel rate of 300 mm/min on a stainless-steel plate of 700 g/25 mm or more;
a melt viscosity at 160°C of 10,000 mPa·s or less;
a melt viscosity at 140°C of 15,000 mPa·s or less; and
a softening point temperature of 100°C or less,
wherein the content of the metallocene-based olefin resin in the hot-melt pressure-sensitive adhesive is from 8 wt% to 0.1 wt%,
and wherein the content of the hydrocarbon-based tackifier in the hot-melt pressure-sensitive adhesive is from 60 wt% to 30 wt%.

2. The hot-melt pressure-sensitive adhesive according to claim 1, wherein the Ziegler-Natta-based olefin resin is free of an aromatic structure.

3. The hot-melt pressure-sensitive adhesive according to claim 1, wherein a content of the Ziegler-Natta-based olefin resin in the hot-melt pressure-sensitive adhesive is from 70 wt% to 20 wt%.

4. The hot-melt pressure-sensitive adhesive according to claim 1, wherein the metallocene-based olefin resin is free of an aromatic structure.

5. The hot-melt pressure-sensitive adhesive according to claim 1, wherein a content of the metallocene-based olefin resin in the hot-melt pressure-sensitive adhesive is from 5 wt% to 1 wt%.

6. The hot-melt pressure-sensitive adhesive according to claim 1, wherein the hydrocarbon-based tackifier is free of an aromatic structure.

7. The hot-melt pressure-sensitive adhesive according to claim 1, wherein a content of the oil component in the hot-melt pressure-sensitive adhesive is from 40 wt% to 5 wt%.

8. The hot-melt pressure-sensitive adhesive according to claim 1, wherein the oil component comprises a paraffin oil.

9. A stretchable laminate, comprising:
an elastomer layer; and
a non-woven fabric layer arranged on at least one side of the elastomer layer,
wherein the stretchable laminate has the hot-melt pressure-sensitive adhesive of claim 1 between the elastomer layer and the non-woven fabric layer.

10. The stretchable laminate according to claim 9, wherein a main component of the elastomer layer comprises an elastomer resin and the elastomer resin comprises an olefin-based elastomer.

11. The stretchable laminate according to claim 10, wherein the olefin-based elastomer has a density of from 0.890 g/cm³ to 0.830 g/cm³.

12. The stretchable laminate according to claim 10, wherein the olefin-based elastomer has a MFR at 230°C and 2.16 kgf of from 1.0 g/10 min to 25.0 g/10 min.

13. An article, comprising the stretchable laminate of claim 9.

## Patentansprüche

1. Haftschmelzklebstoff, umfassend:
ein Olefinharz auf Ziegler-Natta-Basis;
ein Olefinharz auf Metallocen-Basis;
ein klebrigmachendes Mittel auf Kohlenwasserstoff-Basis; und
eine Ölkomponente,
wobei der Haftschmelzklebstoff aufweist:
eine Klebkraft bei 23°C, einem Schälwinkel von 90° und einer Schälgeschwindigkeit von 300 mm/min auf einem Polypropylen-Spinnvliesstoff mit einem Flächengewicht von 19 g /m² von 200 g/25 mm oder mehr;
eine Klebkraft bei 23°C, einem Schälwinkel von 90° und einer Schälgeschwindigkeit von 300 mm/min auf einer Edelstahlplatte von 700 g/25 mm oder mehr;
eine Schmelzviskosität bei 160°C von 10000 mPa·s oder weniger;
eine Schmelzviskosität bei 140°C von 15000 mPa·s oder weniger; und
eine Erweichungspunkttemperatur von 100°C oder weniger,
wobei der Gehalt des Olefinharzes auf Metallocen-Basis in dem Haftschmelzklebstoff von 8 Gew.-% bis 0,1 Gew.-% beträgt,
und wobei der Gehalt des klebrigmachenden Mittels auf Kohlenwasserstoff-Basis in dem Haftschmelzklebstoff von 60 Gew.-% bis 30 Gew.-% beträgt.

2. Haftschmelzklebstoff nach Anspruch 1, wobei das Olefinharz auf Ziegler-Natta-Basis frei von einer aromatischen Struktur ist.

3. Haftschmelzklebstoff nach Anspruch 1, wobei ein Gehalt des Olefinharzes auf Ziegler-Natta-Basis in dem Haftschmelzklebstoff von 70 Gew.-% bis 20 Gew.-% beträgt.

4. Haftschmelzklebstoff nach Anspruch 1, wobei das Olefinharz auf Metallocen-Basis frei von einer aromatischen Struktur ist.

5. Haftschmelzklebstoff nach Anspruch 1, wobei ein Gehalt des Olefinharzes auf Metallocen-Basis in dem Haftschmelzklebstoff von 5 Gew.-% bis 1 Gew.-% beträgt.

6. Haftschmelzklebstoff nach Anspruch 1, wobei das klebrigmachende Mittel auf Kohlenwasserstoff-Basis frei von einer aromatischen Struktur ist.

7. Haftschmelzklebstoff nach Anspruch 1, wobei ein Gehalt der Ölkomponente in dem Haftschmelzklebstoff von 40 Gew.-% bis 5 Gew.-% beträgt.

8. Haftschmelzklebstoff nach Anspruch 1, wobei die Ölkomponente ein Paraffinöl umfasst.

9. Dehnbares Laminat, umfassend:
eine Elastomerschicht; und
eine Vliesstoffschicht, die auf mindestens einer Seite der Elastomerschicht angeordnet ist,
wobei das dehnbare Laminat den Haftschmelzklebstoff nach Anspruch 1 zwischen der Elastomerschicht und der Vliesstoffschicht aufweist.

10. Dehnbares Laminat nach Anspruch 9, wobei eine Hauptkomponente der Elastomerschicht ein Elastomerharz umfasst und das Elastomerharz ein Elastomer auf Olefin-Basis umfasst.

11. Dehnbares Laminat nach Anspruch 10, wobei das Elastomer auf Olefin-Basis eine Dichte von 0,890 g/cm³ bis 0,830 g/cm³ aufweist.

12. Dehnbares Laminat nach Anspruch 10, wobei das Elastomer auf Olefin-Basis eine MFR bei 230°C und 2,16 kgf von 1,0 g/10 min bis 25,0 g/10 min aufweist.

13. Gegenstand, umfassend das dehnbare Laminat nach Anspruch 9.

## Revendications

1. Adhésif thermofusible sensible à la pression, comprenant:
une résine oléfinique à base de Ziegler-Natta;
une résine oléfinique à base de métallocène;
un agent tackifiant à base d'hydrocarbone; et
un composant huileux,
dans lequel l'adhésif thermofusible sensible à la pression présente:
un pouvoir adhésif à 23°C, un angle de pelage de 90° et une vitesse de pelage de 300 mm/min sur tissu non tissé filé-lié direct de polypropylène ayant un poids de base de 19 g/m² de 200 g/25 mm ou plus;
un pouvoir adhésif à 23°C, un angle de pelage de 90° et une vitesse de pelage de 300 mm/min sur plaque en acier inoxydable de 700 g/25 mm ou plus;
une viscosité à l'état fondu à 160°C de 10000 mPa•s ou moins;
une viscosité à l'état fondu à 140°C de 15000 mPa•s ou moins; et
une température de point de ramollissement de 100°C ou moins,
dans laquelle la teneur en résine oléfinique à base de métallocène dans l'adhésif thermofusible sensible à la pression va de 8% en poids à 0,1% en poids,
et dans laquelle la teneur en agent tackifiant à base d'hydrocarbone dans l'adhésif thermofusible sensible à la pression va de 60% en poids à 30% en poids.

2. Adhésif thermofusible sensible à la pression selon la revendication 1, dans lequel la résine oléfinique à base de Ziegler-Natta est exempte d'une structure aromatique.

3. Adhésif thermofusible sensible à la pression selon la revendication 1, dans lequel la teneur en résine oléfinique à base de Ziegler-Natta dans l'adhésif thermofusible sensible à la pression va de 70% en poids à 20% en poids.

4. Adhésif thermofusible sensible à la pression selon la revendication 1, dans lequel la résine oléfinique à base de métallocène est exempte d'une structure aromatique.

5. Adhésif thermofusible sensible à la pression selon la revendication 1, dans lequel la teneur en résine oléfinique à base de métallocène dans l'adhésif thermofusible sensible à la pression va de 5% en poids à 1% en poids.

6. Adhésif thermofusible sensible à la pression selon la revendication 1, dans lequel l'agent tackifiant à base d'hydrocarbone est exempt d'une structure aromatique.

7. Adhésif thermofusible sensible à la pression selon la revendication 1, dans lequel la teneur en composant huileux dans l'adhésif thermofusible sensible à la pression va de 40% en poids à 5% en poids.

8. Adhésif thermofusible sensible à la pression selon la revendication 1, dans lequel le composant huileux comprend une huile de paraffine.

9. Laminé extensible, comprenant:
une couche d'élastomère; et
une couche de tissu non tissé disposée sur au moins un côté de la couche d'élastomère,
dans lequel le laminé extensible comprend l'adhésif thermofusible sensible à la pression selon la revendication 1 entre la couche d'élastomère et la couche en tissu non tissé.

10. Laminé extensible selon la revendication 9, dans lequel un composant principal de la couche d'élastomère comprend une résine d'élastomère et la résine d'élastomère comprend un élastomère à base d'oléfine.

11. Laminé extensible selon la revendication 10, dans lequel l'élastomère à base d'oléfine présente une densité allant de 0,890 g/cm³ à 0,830 g/cm³.

12. Laminé extensible selon la revendication 10, dans lequel l'élastomère à base d'oléfine présente un MFR à 230°C et 2,16 kgf allant de 1,0 g/10 min à 25,0 g/10 min.

13. Article comprenant le laminé extensible selon la revendication 9.
